Europäisches Patentamt

(19) European Patent Office

Office européen des brevets

(11) Publication number: **0 009 894**
**B1**

# EUROPEAN PATENT SPECIFICATION

(45) Date of publication of patent specification: **06.01.82**

(21) Application number: **79301843.3**

(22) Date of filing: **07.09.79**

(51) Int. Cl.³: **B 01 J 29/38, C 07 C 2/66, C 07 C 6/12, C 07 C 5/27, C 07 C 1/20**

(54) Regeneration of zeolite catalyst.

(30) Priority: **11.09.78 US 941606**
**11.09.78 US 941607**

(43) Date of publication of application:
**16.04.80 Bulletin 80/8**

(45) Publication of the grant of the European patent:
**06.01.82 Bulletin 82/1**

(84) Designated Contracting States:
**BE DE FR GB IT NL**

(56) References cited:
**DE - A - 2 053 010**
**DE - B - 1 276 009**
**FR - A - 2 377 226**
**US - A - 4 080 395**

(73) Proprietor: **MOBIL OIL CORPORATION**
**150 East 42nd Street**
**New York New York 10017 (US)**

(72) Inventor: **Haag, Werner Otto**
**38 Pine Knoll Drive**
**Lawrenceville New Jersey 08048 (US)**
Inventor: **Kaeding, Warren William**
**700 Mountain Avenue**
**Westfield New Jersey 07090 (US)**
Inventor: **Olson, David Harold**
**11 Morningside Drive**
**Pennington New Jersey 08534 (US)**
Inventor: **Rodenwald, Paul Gerhard**
**4 Merritt Lane**
**Rocky Hill New Jersey 08553 (US)**

(74) Representative: **Cooper, John Anthony**
**Mobil Court 3 Clements Inn**
**London WC2A 2EB (GB)**

Courier Press, Leamington Spa, England.

Regeneration of zeolite catalyst

This invention relates to a method for regeneration of an aged crystalline zeolite catalysts by treatment with hydrogen under particular conditions.

During the course of converting organic compounds involving reactions such as alkylation, disproportionation, isomerization, cracking, polymerization, aromatization, etc. employing a catalyst comprising a crystalline aluminosilicate zeolite having a silica to alumina mole ratio of at least 12, a constraint index between 1 and 12 and particular xylene sorption characteristics conducive to para selectivity, it has been observed that the catalyst aged during conversion. Aging has been attributed to the deposition of coke which forms during the conversion reaction and covers the active sites of the catalyst, thereby reducing its activity which in turn results in lower conversion.

One method heretofore employed for regeneration of such catalysts to restore high product conversion has been to:

(1) purge the system of organic compounds;

(2) lower the temperature to about 700°F;

(3) cautiously add air/nitrogen mixtures which contain about 1 percent oxygen at a rate to prevent the temperature, usually a hot zone progressing from top to bottom of the catalyst bed, from exceeding about 482°C as the coke is burned to form CO and $H_2O$;

(4) enrich the oxygen concentration gradually after the bulk of the coke has been burned until it reaches a level of from about 3 to 20 percent oxygen, the latter oxygen content corresponding to air, taking care not to exceed a temperature of about 482°C;

(5) purge the system with nitrogen to remove any residual oxygen; and

(6) begin reaction by restoring conditions and feeds.

This method requires purging the reactor before and after coke burning. It is also necessary very carefully to monitor the temperature throughout the entire catalyst bed to prevent excessive temperatures which could permanently damage and deactivate the catalyst. This method has the further disadvantage of removing all coke from the catalyst, necessitating, where desired, the redeposition of a controlled amount of coke on the catalyst surface to impart selectivity characteristics thereto as, for example, in the selective production of paraxylene by methylation of toluene described in U.S. 4,001,346.

Other methods previously proposed for regenerating such catalysts are described in DE—AS 1,276,009 in which coked catalyst is restored to substantially initial activity by hydrogen treatment at 315 to 538°C, and DE—OS 2,053,010 in which coked catalyst is treated before reuse with hydrogen at 350 to 650°C. Neither document, however, refers to the presence of selectivating coke on the catalyst, presumably since it was thought that hydrogen regeneration would result in removal of all coke from the catalyst.

The present invention is based upon the surprising observation that hydrogen treatment of a zeolite catalyst which has been precoked to increase its selectivity and which has become further coked by its use in an organic conversion, results in removal of the coke deposited by use and not of selectivating coke. Thus, this method eliminates not only the undesirable purging steps required in the regeneration method using oxygen and enables regeneration to be carried out in a single processing step, but also the coke-redeposition step associated with that method.

According to the present invention there is provided a method of regenerating a catalyst bearing a deposit of coke as a consequence of its use in an organic compound conversion process, said catalyst comprising a crystalline zeolite having a silica to alumina ratio of at least 12, a constraint index of 1 to 12, a xylene sorption capacity of at least 1 g./100 g. of zeolite and an o-xylene sorption time for 30 percent of said capacity of greater than 10 minutes, said sorption capacity and time being measured at 120°C and a xylene pressure of 600 ± 106 Pa, and said catalyst having been modified before use by controlled deposition thereon of at least 1 weight percent of selectivating coke, the method comprising exposing the catalyst to a hydrogen-containing atmosphere at from 425 to 650°C and from 101 to 13890 kPa to remove the coke deposited as a consequence of use of the catalyst in the conversion process but not the selectivating coke.

The zeolite is modified before use in the organic compound conversion process by controlled deposition thereon of at least 1 weight percent of coke. Coke deposition may be effected by contacting the catalyst with a thermally decomposable organic compound at conditions including a temperature above the decomposition temperature of that compound but below 648°C and a hydrogen/compound ratio between 0 and 1, said conditions being thereby collectively more severe than those prevailing during use of the catalyst.

Preferably the catalyst is regenerated at a pressure of 170 to 6996 kPa, still more preferably at a pressure of 460 to 3204 kPa. In desirable embodiments the hydrogen atmosphere contacts the catalyst at a weight hourly space velocity of 0.25 to 10, and the duration of the regeneration is 1 to 48 hours, preferably 2 to 30 hours.

The thermally decomposable organic compound may be an organic compound subjected to the conversion process, a frequently used compound being toluene.

The zeolite advantageously has a crystal size greater than 0.5 micron, and the preferred zeolite is ZSM-5. In addition to the controlled deposition thereon of selectivating coke, the catalyst may also be modified before use by combination therewith of a difficultly reducible oxide, that is an oxide of antimony, boron, magnesium and/or phosphorus, in an amount of 0.5 to 40 weight percent. In practice the zeolite is usually composited with a binder, such as alumina.

The hydrogen regeneration which characterizes the invention applies particularly to conversion processes such as alkylation of an aromatic hydrocarbon, disproportionation of an alkyl aromatic hydrocarbon, xylene isomerisation or conversion of lower monohydric alcohols and or their corresponding ethers to an olefin-rich hydrocarbon mixture.

The catalyst undergoing regeneration in accordance with the method described herein comprises a crystalline aluminosilicate zeolite which is a member of a novel class of zeolites exhibiting some unusual properties. These zeolites are in particular defined as those possessing a constraint index of 1 to 12 and the xylene sorption characteristics hereinabove set forth. The significance of that index and those characteristics, and techniques for their measurement, are fully set forth in many of our prior specifications, for instance DE—OS 2,714,239 (which is concerned with catalysts the zeolite in which is further characterized by an alpha value of 2 to 5000).

The class of zeolites defined herein is exemplified by ZSM-5, ZSM-11, ZSM-12, ZSM-35 and ZSM-38, described respectively in U.S. specifications 3,702,886, 3,709,979, 3,832,449, 4,016,245 and 4,046,859.

These zeolites, when prepared in the presence of organic cations, are catalytically inactive, possibly because the intracrystalline free space is occupied by organic cations from the forming solution. They may be activated by heating in an inert atmosphre at 37.8°C (100°F) for one hour, for example, followed by base exchange with ammonium salts followed by calcination at 537°C in air. The presence of organic cations in the forming solution may not be absolutely essential to their formation; however, the presence of these cations does appear to favor the formation of this special type of zeolite. More generally, it is desirable to activate the zeolite by base exchange with ammonium salts followed by calcination in air at about 537°C for from about 15 minutes to about 24 hours. The base exchange should reduce the alkali metal content of the zeolite to below 1.5 weight percent, and replacing ions can, in addition to hydrogen/ammonium, be selected from groups IB to VIII of the Periodic Table including, by way of example nickel, copper, zinc, palladium, calcium or rare earth. Preferably at least 50, and desirably more than 75, percent of the cationic sites of the zeolites are occupied by hydrogen ions.

In a preferred aspect of this invention, the zeolites hereof are selected as those having a crystal framework density, in the dry hydrogen form, of not substantially below about 1.6 grams per cubic centimeter, as also set forth in the aforementioned DT—OS 2,714,239.

The crystal size of the as synthesized zeolite is generally within the approximate range of .01 to 40 microns. Preferably, the crystal size of the zeolite employed in the process of the invention is greater than about 0.5 micron, usually in the approximate range of 1—20 microns and particularly in the range of 1—6 microns. With the use of crystals within such size range, distinctly higher selectivity for production of desired product has been observed as compared with comparable use of smaller size crystals.

In practice the zeolite is frequently incorporated in another material resistant to the temperature and other conditions employed in the process, again as fully described in DT—OS 2,714,239.

Modification of the zeolite with a difficulty reducible oxide or oxides can readily be effected by contacting the zeolite with a solution of an appropriate compound of the element to be introduced, followed by drying and calcining to convert the compound to its oxide form. Specific techniques for introducing the oxides of phosphorus and magnesium, which is an advantageous embodiment are present simultaneously, are described in aforementioned DT—OS 2,714,239.

Boron oxide is also an effective modifying component. Representative boron-containing compounds include boric acid, trimethylborate, boron hydride, boron oxide, boron sulfide, butyl-boron dimethoxide, butylboronic acid, dimethyl-boric anhydride, hexamethylborazine, phenyl-boric acid, triethylborane, tetramethylammonium borohydride, triphenyl boron and allylborate.

Reaction of the zeolite with the boron compound is effected by contacting the zeolite with such compound. Where the treating boron compound is a liquid, such compound can be in solution in a solvent at the time contact with the zeolite is effected. Any solvent relatively inert with respect to the treating compound and the zeolite may be employed. Suitable solvents include water and aliphatic, aromatic or alcoholic liquids. Where the boron-containing compound is, for example, trimethylborate, a hydrocarbon solvent such as n-octane may be employed. The boron-containing compound may be used without a solvent, i.e., may be used as a neat liquid. Where the boron-containing compound is in the gaseous phase, such as where gaseous diborane is employed, the treating compound can be used by itself or can be used in admixture with a gaseous diluent inert to the boron-containing compound and the zeolite such as nitrogen or helium or with an organic solvent, such as octane.

Prior to reacting the zeolite with the boron-containing compound, the zeolite may be dried. Drying can be effected in the presence of air. Elevated temperatures may be employed. However, the temperature should not be such that the crystal structure of the zeolite is destroyed.

Heating of the boron-containing catalyst subsequent to preparation and prior to use is also preferred. The heating can be carried out in the presence of oxygen, for example, air. Heating can be at a temperature of about 150°C. However, higher temperatures, i.e., up to about 500°C are preferred. Heating is generally carried out for 3—5 hours but may be extended to 24 hours or longer. While heating temperatures above about 500°C can be employed, they are not necessary. At temperatures of about 1000°C, the crystal structure of the zeolite tends to deteriorate.

The amount of boron incorporated with the zeolite should be at least 0.5 percent by weight. However, it is preferred that the amount of boron in the zeolite be at least 1 percent by weight when the same is combined with a binder, e.g., 35 weight percent of alumina. The amount of boron can be as high as 20 percent by weight or more depending on the amount and type of binder present. Preferably, the amount of boron added to the zeolite is between 1.5 and 10 percent by weight. Without being limited by any theoretical considerations, it is contemplated that boron is actually present in the zeolite in an oxidized state, such as $B_2O_3$.

Antimony oxide may also be employed as a modifying component. The antimony oxide is present as $Sb_2O_3$ alone or in admixture with other antimony oxides with or without metallic antimony or other antimony compounds being present. In all instances, regardless of the particular state of oxidation of the antimony, its content with respect to the zeolite is computed as if it were present as $Sb_2O_3$. Generally, the amount of $Sb_2O_3$ in the composite catalyst will be between about 6 and about 40 weight percent and preferably between about 10 and about 35 weight percent. Antimony derivations which may be used include: the hydrides $SbH_3$; the halides $MX_3$, $MX_5$($M=Sb$, $X=F$, Cl, Br, I); organic alkyl and aryl stibines and their oxides $R_3Sb$, $R_5Sb$, $R_xSb=O$($R$-alkyl or aryl); halogen derivatives $RSbX_2$, $R_2SbX$, $RSbX_4$, $R_2SbX_3$, $R_3SbX_2$, $R_4SbX$; the acids $H_3SbO_3$, $HSbO_2$, $HSb(OH)_6$; organic acids such as $RSbO(OH)_2$, $R_2SbO \cdot OH$, all with R and X defined as above noted. Also included are organic ethers such as $R_2SbOSbR_2$; esters and alcoholates such as $Sb(OOCCH_3)_3$, $Sb(OC_4H_9)_3$, $Sb(OC_2H_5)_3$, $Sb(OCH_3)_3$; and antimonyl salts as $(SbO)SO_4$, $(SbO)NO_3$, $K(SbO)C_4H_4O_6$, $NaSbO_2 \cdot 3H_2O$.

Enhancement of the above crystalline aluminosilicate zeolite catalyst modified by combination therewith of one or more of the above difficultly reducible oxides may further be obtained by steaming and/or precoking thereof. Steaming is effectively carried out using an atmosphere containing from 5 to 100 percent steam at a temperature of from 250 to 1000°C for a period of between 0.5 and 100 hours under pressures ranging from sub-atomspheric to several hundred atmospheres.

Controlled precoking i.e., selectivation, may be carried out separately or prior to the specified steam treatment by exposing the catalyst to a thermally decomposable organic compound, e.g., toluene, under high severity conditions at a temperature in excess of the decomposition temperature of said compound, generally greater than 537°C, but less than about 648°C, at a hydrogen to organic compound mole ratio between 0 and 1 to deposit the desired quantity of coke thereon, generally an amount of at least 1 percent by weight.

For toluene and organic compounds of similar reactivity, the temperature employed is greater than 537°C. With organic compounds that are more readily decomposable than toluene, precoking can be carried out at temperatures of less than 537°C. With the use of higher temperatures in the aforenoted range the presence of hydrogen has not been found necessary. With temperatures of less than about 593°C, preferably some hydrogen, generally at least 0.2 mole of hydrogen per mole of organic compound is desirable.

Organic materials, thermally decomposable under the above temperature conditions to provide coke deposition, encompass a wide variety of compounds including by way of example, hydrocarbons, such as paraffinic, cycloparaffinic, olefinic, cycloolefinic and aromatic; oxygen-containing organic compounds such as alcohols, aldehydes, ethers, ketones and phenols; heterocyclics such as furans, thiophenes, pyrroles and pyridines. Usually, it is contemplated that a thermally decomposable hydrocarbon, such as an alkylsubstituted aromatic, will be the source of coke and most preferably toluene. In the latter case, toluene is initially brought into contact under conditions of temperature and hydrogen concentration amenable to rapid coke formation.

The amount of coke deposited on the catalyst, prior to conducting conversion therewith, will ordinarily be at least 1 weight percent. Generally, the amount of coke deposited will not exceed about 60 weight percent. The optimum amount of coke employed will depend among other variables on the crystal size of the aluminosilicate zeolite used and the nature of the catalyst binder, if any, employed.

It has been found that as a general rule, the smaller the zeolite crystal size, the greater the amount of coke deposition required to achieve comparable results. Thus, on a binder-free basis, it has been observed that with the use of small zeolite crystals, e.g. in the range of .02 to .05 micron size, greater than 20 weight percent of coke deposition was required to obtain results comparable to those obtained with larger zeolite crystals, e.g. in the range of 1 to 2 microns, having approximately 4 weight percent of coke deposited thereon.

The organic compound conversion in which the above described crystalline aluminosilicate zeolite catalysts are employed, prior to regeneration, include those reactions wherein coke is inherently deposited on the catalyst as a consequence of the conversion taking place. Typical conversion processes, given by way of example, include the methylation of toluene described in U.S. 3,965,207; 3,965,208; 3,965,209; 3,965,210; 4,001,346, 4,002,697 and 4,002,698; disproportionation of toluene to produce benzene and xylenes rich in the para isomer described in U.S. 4,011,276; 4,016,219 and 4,097,543; isomerization of xylenes described in U.S. 3,856,872; hydrocarbon conversion described in U.S. 3,790,471; alkylation of aromatic hydrocarbons with olefins described in U.S. 3,751,504; 3,751,506; 3,755,483; 3,962,364 and 4,016,218; conversion of lower monohydric alcohols and their ethers, such as methanol and dimethyl ether to hydrocarbon mixtures rich in olefins described in U.S. 3,911,041 and 3,979,472; alkylation of olefins described in U.S. 3,906,054; ethylation of toluene or ethylbenzene to selectively produce the para ethyl derivative thereof described in U.S. 4,086,287 and selective production of para dialkyl substituted benzenes described in U.S. 4,117,026; and selective production of para-xylene described in U.S. 4,080,395.

In accordance with the regeneration method of the present invention, the aged catalyst resulting from use in catalyzing an organic compound conversion of the type indicated hereinabove and containing coke in an amount generally between 0.5 and 5 weight percent, in excess of any prior coke deposited to selectivate the catalyst, is exposed to an atmosphere rich in hydrogen. While the presence of inert contaminants, e.g., nitrogen, may be tolerated, it is generally preferred that the regeneration atmosphere consist essentially of hydrogen. The regeneration conditions are important and critical to the success of the operation. Generally, a temperature between 425 and 650°C and preferably between 500 and 600°C will be employed, with the pressure being between 170 and 6996 kPa and preferably between 446 and 3204 kPa.

It is of interest to note that any coke which was deposited during the selectivation procedure, to obtain a catalyst which provides higher selectivities to a para oriented product, is essentially not removed by the hydrogen regeneration treatment described herein. Without being limited by any theory, it appears as if another and different type of coke is deposited during various conversions of organic compounds such as alkylation, disproportionation, isomerization, cracking, oligomerization, aromatization, etc. It is this latter coke which is selectively removed by hydrogen treatment under the conditions specified, essentially restoring the initial catalyst activity while maintaining the desired high selectivity. Such is not possible when oxygen-containing gases are used in which instance all of the coke is removed indiscriminately.

The amount and nature of the binder composited with the crystalline aluminosilicate zeolite also has been found to have a marked effect on the amount of coke deposition required to obtain the desired selectivity characteristics. Thus, while binder-free zeolite of 1 to 2 micron size afforded high paraxylene selectivity with about 4 weight percent of coke deposited thereon, comparable use of a composite of such zeolite (65 percent) and alumina (35 percent) required an average of about 22 weight percent of coke deposition. With the use of small zeolite crystals (.02 to .05 micron) and alumina binder (35 percent), it is contemplated that 40 percent or more coke deposition would be required to obtain the desired high paraxylene selectivity. Also, increase in alumina content of the zeolite composite, which desirably is in the form of an extrudate, would be expected to require increased coke deposition to obtain comparable high selectivity. With the use of other binders, such as clay or silica, it is anticipated that the amount of coke required for comparable results may be somewhat less than in the case where alumina is the sole binding material.

Figure 1 of the drawing depicts selectivity for para ethyltoluene and conversion of toluene with time on stream during alkylation of toluene with ethylene over a coke-selectivated crystalline aluminosilicate zeolite catalyst of the type used in the present invention.

Figure 2 of the drawing depicts selectivity for paraxylene and conversion of toluene with time on stream during disproportionation of toluene over a coke-selectivated crystalline aluminosilicate zeolite catalyst of the type used in the present invention.

The following examples illustrate the invention and describe four series of experiments (examples 1—7, 8—10, 11—14 and 15—17) to demonstrate its advantages.

Example 1

ZSM-5 zeolite in the hydrogen form (60.5 grams) having a crystal size of about 2 microns, containing 35 weight percent alumina binder, in the form of 1/16″ extrudate, was steamed at 600°C for 1 hour. The steamed material was then impregnated with a solution of 38.7 grams of diammonium acid phosphate in 100 ml of water, dried and calcined at 500°C overnight in an open dish. The resulting product was cooled and impregnated overnight with a solution of 195 grams of magnesium acetate tetrahydrate in 133 ml of water, dried and calcined at 500°C for about 19 hours. The final catalyst contained 4.93 percent magnesium and 3.48 percent phosphorus by weight.

Example 2

Alkylation of toluene with ethylene was carried out in the presence of hydrogen and the catalyst of Example 1. The conditions used for alkylation included a temperature of 425°C, a pressure of 791 kPa, toluene/ethylene/-hydrogen feed WHSV of 29.3/1.16/.24 with the mole ratio of toluene/ethylene/hydrogen being 7.7/1/3. Initial run performance results are summarized in Table 1 below.

TABLE I

| Run No. | Stream Time Hours | Conversion, % | | p-ET, % | | Av. Selectivity % | |
| | | Toluene | $C_2H_4$ | Total ET [a] | p+m–ET [a] | Gas [b] | Other Liquid [c] |
|---|---|---|---|---|---|---|---|
| 1 | 22 | 10.5–10.1 | 85–77 | 96.1 | 94.1 | 1.1 | 4.8 |
| B. Selectivate 21 hrs. | | | | | | | |
| 2 | 25 | 5.2–3.7 | 46–34 | 99.9 | 95.6 | 1.6 | 2.8 |
| C. Regenerate $H_2$ 2 hrs. | | | | | | | |
| 3 | 21 | 5.0–2.5 | 46–23 | 99.9 | 95.5 | 2.2 | 2.3 |
| C. Regenerate $H_2$ 20 hrs. | | | | | | | |
| 4 | 6 | 4.7–4.9 | 44–46 | 99.9 | 96.0 | 1.7 | 2.3 |
| C. Regenerate $H_2$ 17 hrs. | | | | | | | |
| 5 | 7 | 4.9–3.6 | 47–34 | 99.9 | 95.7 | 1.6 | 2.7 |
| C. Regenerate $H_2$ 16 hrs. | | | | | | | |
| 6 | 7 | 4.9–3.6 | 50–34 | 99.9 | 96.0 | 1.6 | 2.4 |
| D. Regenerate Air 13 hrs. | | | | | | | |
| 7 | 2 | 10.4 | 87–85 | 96.9 | 92.0 | 2.0 | 6.0 |
| C. Selectivate 10.75 hrs. | | | | | | | |

0 009 894

TABLE I (Continued)

| Run No. | Stream Time Hours | Conversion, % | | p-ET, % | Av. Selectivity % | | |
| | | Toluene | $C_2H_4$ | Total ET [a] | p+m–ET [a] | Gas [b] | Other Liquid [c] |
| --- | --- | --- | --- | --- | --- | --- | --- |
| 8 | 20 | 8.7–6.8 | 73–56 | 99.8 | 96.7 | .9 | 2.5 |
| 9 | 117 [d] | 8.7–5.9 | 73–44 | 99.7 | 97 | .8 | 2.2 |
| | C. Regenerate $H_2$ 4 hrs. | | | | | | |
| 10 | 18 | 8.3–7.6 | 66–61 | 99.7 | 94.1 | 2.3 | 3.6 |

ET - Ethyltoluene

(a)    No ortho isomer produced

(b)    $C_1$–$C_4$ paraffins and olefins

(c)    Benzene, ethylbenzene and p-xylene primarily

(d)    450°C after first 20 hrs

The maximum toluene conversion, based on the amount of ethylene under the above conditions, was 13 percent. Approximately an 80 percent conversion of starting material was achieved in 22 hours of operation with a high selectivity to ethyltoluene and a very high amount of the para isomer in the ethyltoluene product.

## Example 3

In order to increase the proportion of para isomer in the ethyltoluene ever further, the catalyst was treated with a toluene/hydrogen feed mixture WHSV 29.3/0.8 with the mole ratio of toluene/hydrogen being 7.7/1 at 600°C and 791 kPa pressure of 21 hours to deposit coke thereon. This treatment is referred to as the "selectivation" procedure. As will be seen by reference to Run 2 of the above Table, the amount of p-isomer in the ethyltoluene product increased to 99.9 percent. However, the conversion decreased and the catalyst was found to age at a relatively high rate.

## Example 4

In order to restore the catalyst activity, it was necessary to regenerate the catalyst. Hydrogen was used for this purpose at a temperature of 550°C, a pressure of 791 kPa at a weight hourly space velocity (WHSV) of 0.24. After 2 hours of regeneration, the catalyst was again tested for alkylation under the conditions initially used, Run 3 of the above Table. It will be evident that the performance of the catalyst prior to regeneration (Run 2) was substantially restored. Thus, this technique for regeneration of the catalyst did not remove coke deposited during the selectivation procedure, Example 3, enhancing para selectivity but did remove the coke produced during the alkylation reaction which led to reduced catalyst alkylation ability. The initial reactivity of the selectivated catalyst was completely restored.

## Example 5

The catalyst was regenerated with hydrogen in a manner similar to that described above and again tested for alkylation performance, Runs 4, 5 and 6 of the Table. It will be seen that the initial performance of the highly selectivated catalyst was subsantially restored in every case' to give 99.9% of the p-ethyltoluene in the ethyltoluene product.

## Example 6

After Run 6, Table 1, the catalyst was regenerated with air. In conducting such operation, the reactor was purged with nitrogen, and then air, initially diluted with nitrogen, was carefully and gradually admitted at 450°C at such a rate to avoid a temperature exotherm. The temperature was gradually raised to 550°C and the nitrogen flow diminished until pure air was flowing. The time of calcination was measured for conditions where pure air was used at 550°C. It will be evident from a comparison of the results of Runs 1 and 7 of the above Table that the described treatment resulted in alkylation performance substantially the same as that achieved with the original catalyst. In this case, it was apparent that all of the coke was removed, including that deposited by the selectivation procedure, and that produced during the alkylation reaction.

## Example 7

The catalyst, after the conclusion of the operation described in Example 6, was selectivated by treatment with toluene and hydrogen for 10.75 hours and then tested for alkylation performance as described hereinabove. It will be seen from the results of Runs 8 and 9 of Table 1 that very high concentrations of para isomer in the ethyltoluene product, i.e., 99.7—99.8%, were obtained at relatively high conversions and selectivity. Regeneration of the catalyst with hydrogen for 4 hours under the conditions specified in Example 4 substantially restored the catalyst activity to its original value ("C" in Table I), as will be evident from a comparison of the results of Runs 8 and 10 of Table I.

## Example 8

Alkylation of ethylbenzene with ethylene to produce diethylbenzene was carried out with the catalyst of Example 1. Such catalyst was calcined in air prior to use in a manner similar to that described in Example 6. Conditions of alkylation used included a temperature of 425°C, a pressure of 791 kPa, an ethylbenzene/ethylene/hydrogen feed WHSV of 29.71/1.16/ .24 with the mole ratio of ethylbenzene/ ethylene/hydrogen being 6.8/1/3.

After Runs 1 and 2, prior to Runs A and B, Table III the catalyst was selectivated by treating it with a mixture of toluene-hydrogen WHSV 30/.08 at 600°C, 791 kPa for a period of about 11 hours.

The conditions used to selectively regenerate with hydrogen before runs A and B was 550°C, 791 kPa and WHSV of pure hydrogen of 0.24. Results are summarized in Table III below.

TABLE III

| Run No. | Gas Diluent | Stream Time Hours | Conversion, % | | p-DEB Total DEB (a) % | Selectivity p+m—DEB % |
|---|---|---|---|---|---|---|
| | | | EB | $C_2H_4$ | | |
| | Regenerate Air | 20 hrs. | | | | |
| 1 | $H_2$ | 50 | 21.9—16.3 (b) | 65—55 | 98.4—98.9 | 71.6—80.0 |
| | Regenerate Air | 17 hrs. | | | | |
| 2 | $N_2$ | 46 | 21.7—12.3 | 56—39 | 98.3—98.9 | 72.1—83.0 |
| | Regenerate Hydrogen | 5 hrs. | | | | |
| A | $H_2$ | 44 | 14.3—3.9 | 54—21 | 99.8—99.9 | 76.8—75.9 |
| | Regenerate Hydrogen | 3 hrs. | | | | |
| B | $N_2$ | 47 | 11.6—2.6 | 31—7 | 99.8—99.7 | 79.8—77.3 |

(a)  None of the ortho isomer could be detected.

(b)  The range shows the result for the first and last hour of the time period tested.

EB = Ethylbenzene, DEB = Diethylbenzene.

It will be seen from Run 1 of the above Table that the initial toluene conversion was 21.9% during the first hour of operation and was reduced to 16.3% during the 50th hour. It is to be noted that the maximum theoretical ethylbenzene conversion based on the amount of ethylene in the feed, and assuming that the only reaction was alkylation to diethylbenzene was about 14.8 percent. Under the conditions of reaction employed, however, ethylbenzene was also consumed by disproportionation to yield primarily benzene and para-diethylbenzene accounting for the additional conversion. As will further be evident, selectivity to the para isomer in the diethylbenzene product was very high, i.e., 98.4—98.9 percent.

### Example 9

In a manner similar to Example 8, the alkylation and disproportionation of ethylbenzene was carried out with the same catalyst used in Example 8 but using a nitrogen diluent after regeneration of the catalyst in air. The conditions of temperature and pressure were the same as those used in the previous example. The ethylbene/ethylene/nitrogen feed WHSV was 29.71/1.16/3.47 with the mole ratio of ethylbenze/ethylene/nitrogen being 6.8/1/3.

The results obtained are summarized in Table III, Run 2. It will be seen that the original catalyst activity was substantially restored but that catalyst aging was significantly greater with the nitrogen diluent as compared with the hydrogen diluent.

### Example 10

In a manner similar to that described in Examples 8 and 9, except that hydrogen rather than air was used for the regenerations, catalyst performance was tested for the alkylation of ethylbenzene with ethylene to produce diethylbenzene.

In this example, the catalyst had been selectivated with toluene and hydrogen prior to use, in a manner similar to that described in Example 3.

Results are summarized in Runs A and B of Table III, from which it will be seen that the catalyst was regenerated with hydrogen in an overall manner similar to that observed for regeneration in air. The initial conversion obtained for Run B, i.e., 11 percent was less than that for Run A, i.e., 14.3 percent presumably due to the shorter time period for regeneration.

Thus it can be seen that the coke deposited during the selectivation prior to Run A, Table III, which increased the selectivity to p-DEB to 99.8—99.9% was not removed during the regeneration with hydrogen following Run A, according to the conditions for regeneration described but did significantly increase activity from 3.9% (end of Run A) to 11.6% (beginning of Run B), by removing only the coke deposited during the alkylation reaction.

### Example 11

A sample of HZSM-5 (2.0 grams), characterized by a crystal size of 1—2 microns, in the form of an extrudate with 35 weight percent alumina, was selectivated with coke. Selectivation conditions included passing a stream of toluene and hydrogen over the zeolite for 25 hours at 593°C, 6.5 WHSV, 0.5 $H_2$/toluene and 308 kPa.

### Example 12

The selectivated catalyst of Example 11 was tested for toluene disproportionation by passing a stream of toluene thereover at WHSV of 6.5, a pressure of 2859 KPa and a hydrogen/hydrocarbon ratio of 4 at a temperature of 482°C. At 17 percent conversion, the paraxylene content of the total xylenes produced was 75 percent.

### Example 13

The catalyst used in Example 12 was then tested for ethylation of toluene by contacting with a toluene/ethylene/hydrogen stream at a weight hourly space velocity of 28/1.1/0.24 a temperature of 425°C and a presssure of 791 kPa. At a toluene conversion of 55 percent of theory, there was observed 90 percent of para-ethyltoluene in the ethyltoluenes produced. Toluene conversion dropped to 30 percent after two days on stream.

### Example 14

The catalyst used in Example 13 was then regenerated by treating with hydrogen at a rate of 100cc of hydrogen per minute for 2 hours at 538°C. It was found that the catalyst could be restored to its initial activity and selectivity.

The catalyst then aged during the next six days to 30 percent conversion. A two hour regeneration by treatment with hydrogen under the above conditions did not completely restore the initial catalyst activity. However, increasing the regeneration time to 16 hours restored the activity as did a subsequent 16 hour regeneration. The selectivity to para-ethyltoluene gradually increased from 90 to 93 percent during the 16 days on stream and was not affected by the hydrogen regeneration.

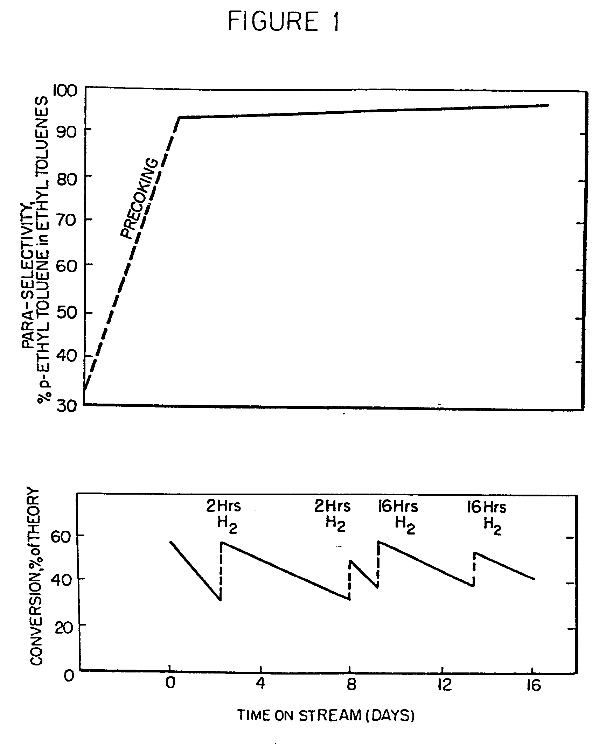

The above results employing hydrogen regeneration are shown graphically in Figure 1.

### Example 15

A sample of HZSM-5 (1.0 g), characterized by a crystal size of 1—2 microns, in the form of an extrudate with 35 weight percent alumina, was selectivated with coke. Selectivation conditions included passing a stream of toluene over the extrudate particles for 112 hours at 566°C, 6.5 WHSV, 0.5 $H_2$/HC and 308 kPa.

### Example 16

The selectivated catalyst of Example 15 was used for toluene disproportionation for six months by passing a stream of toluene thereover at a WHSV of 6.5, $H_2$/HC = 4, and a pressure of 2859 kPa. During this period, catalyst aging was compensated by increasing the temperature from 482°C at the beginning to 485°C at the end of the cycle. The toluene conversion was 24 percent. The paraxylene content of the total xylenes produced was 82 percent at the beginning and 93 percent at the end of the six month period. For comparison, the catalyst prior to coke selectivation gave xylenes containing only 30% p-xylene.

Example 17

The used catalyst of Example 16 was hydrogen regenerated by passing hydrogen thereover at 538°C and 2859 kPa for a total of 68 hours. After this regeneration, the catalyst produced 82 percent paraxylene in total xylenes at 24 percent conversion at a temperature of 482°C, i.e., at the test conditions at the beginning of the catalytic cycle of Example 6.

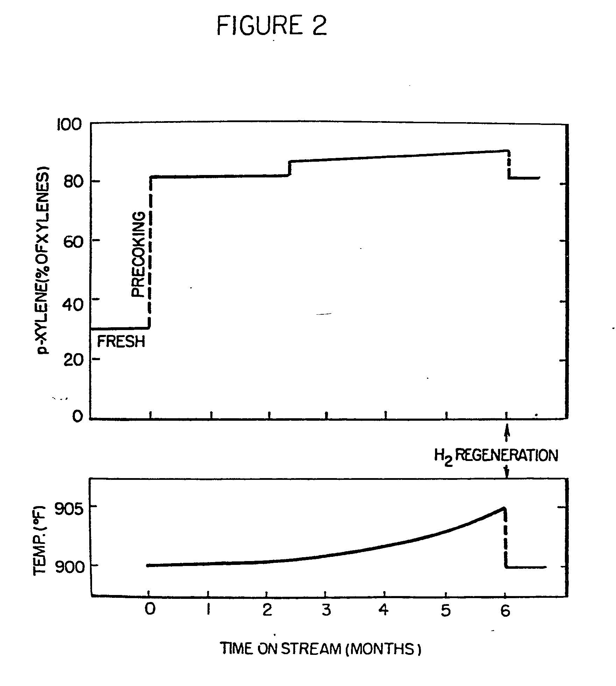

The paraxylene selectivity changes for the catalyst materials of Examples 15, 16 and 17 are summarized in Figure 2.

## Claims

1. A method of regenerating a catalyst bearing a deposit of coke as a consequence of its use in an organic compound conversion process, said catalyst comprising a crystalline zeolite having a silica to alumina ratio of at least 12, a constraint index of 1 to 12, a xylene sorption capacity of at least 1 g./100 g. of zeolite and an o-xylene sorption time for 30 percent of said capacity of greater than 10 minutes, said sorption capacity and time being measured at 120°C and a xylene pressure 600 ± 106 Pa and said catalyst having been modified before use by controlled deposition thereon of at least 1 weight percent of selectivating coke, said method comprising exposing the catalyst to a hydrogen-containing atmosphere at from 425 to 650°C and from 101 to 13890 kPa to remove the coke deposited as a consequence of use of the catalyst in the conversion process but not the selectivating coke.

2. A method according to claim 1, wherein said zeolite has been modified before use by combination therewith of 0.5 to 40 weight percent of an oxide of antimony, boron, magnesium and/or phosphorus.

3. A method according to claim 1 or claim 2, wherein the catalyst is regenerated at a pressure of 170 to 6996 kPa.

4. A method according to any preceding claim, wherein the catalyst is regenerated at a pressure of 460 to 4304 kPa.

5. A method according to any preceding claim, wherein the hydrogen-containing atmosphere contacts the catalyst at a weight hourly space velocity of 0.25 to 10.

6. A method according to any preceding claim, wherein the duration of the regeneration is 1 to 48 hours.

7. A method according to any preceding claim, wherein the deposition of selectivating coke is effected by contacting the catalyst with a thermally decomposable organic compound at conditions including a temperature above the decomposition temperature of that compound but below 648°C and a hydrogen/compound ratio between 0 and 1.

8. A method according to claim 7, wherein said thermally decomposable organic compound is also an organic compound subjected to said conversion process.

9. A method according to claim 8, wherein said compound is toluene.

10. A method according to any preceding claim, wherein the zeolite is ZSM-5.

11. A method according to any preceding claim, wherein the zeolite has a crystal size greater than 0.5 micron.

12. A method according to any preceding claim, wherein the zeolite is composited with a binder, such as alumina.

13. A method according to any preceding claim, wherein said conversion process is alkylation of an aromatic hydrocarbon, disporportionation of an alkyl aromatic hydrocarbon, xylene isomerisation or conversion of lower monohydric alcohols and/or their corresponding ethers into an olefin-rich hydrocarbon mixture.

## Patentansprüche

1. Verfahren zur Regenerierung eines Katalysators, der einen Niederschlag aus Koks infolge seiner Verwendung in einem Verfahren zur Umwandlung von organischen Verbindungen trägt, wobei der Katalysator einen kristallinen Zeolithen mit einem Siliciumdioxid/Aluminiumoxid-Verhältnis von wenigstens 12, einen Zwangsindex von 1 bis 12, eine Xylol-Sorptionskapazität von wenigstens 1 g/100 g des Zeolithen und eine o-Xylol-Sorptionszeit für 30% der betreffenden Kapazität von mehr als 10 min aufweist und die Sorptionskapazität und -zeit bei 120°C und einem Xylol-Druck von 600 ± 106 Pa gemessen sind und der Katalysator vor Verwendung durch geregelte Niederschlagung darauf von wenigstens 1 Gew.-% von selektivierendem Koks modifiziert worden ist, dadurch gekennzeichnet, daß der Katalysator einer wasserstoffhaltigen Atmosphäre von 425 bis 650°C und von 101 bis 13 890 kPa zur Entfernung des Kokses, der infolge der Katalysatorverwendung bei dem Umwandlungsverfahren niedergeschlagen worden ist, jedoch nicht des selektivierenden Kokses ausgesetzt wird.

2. Verfahren nach Anspruch 1, dadurch gekennzeichnet, daß der Zeolith vor Verwendung durch Kombination mit 0,5 bis 40 Gew.-% eines Oxids von Antimon, Bor, Magnesium und/oder Phosphor modifiziert worden ist.

3. Verfahren nach den vorhergehenden Ansprüchen, dadurch gekennzeichnet, daß der Katalysator bei einem Druck von 170 bis 6 996 kPa regeneriert wird.

4. Verfahren nach den vorhergehenden Ansprüchen, dadurch gekennzeichnet, daß der Katalysator bei einem Druck von 460 bis 4 304 kPa regeneriert wird.

5. Verfahren nach den vorhergehenden Ansprüchen, dadurch gekennzeichnet, daß die wasserstoffhaltige Atmosphäre mit dem Katalysator bei einer gewichtsmäßigen stündlichen Raumströmungsgeschwindigkeit von 0,25 bis 10 in Berührung steht.

6. Verfahren nach den vorhergehenden Ansprüchen, dadurch gekennzeichnet, daß die Dauer der Regenerierung 1 bis 48 Stunden beträgt.

7. Verfahren nach den vorhergehenden Ansprüchen, dadurch gekennzeichnet, daß die Niederschlagung von selektivierendem Koks durch Inberührungbringen des Katalysators mit einer thermisch zersetzbaren organischen Verbindung bei Bedingungen einschließlich einer Temperatur oberhalb der Zersetzungstemperatur der betreffenden Verbindung, jedoch unterhalb 648°C, und einem Wasserstoff/Verbindung-Verhältnis zwischen 0 und 1 erzielt wird.

8. Verfahren nach Anspruch 7, dadurch gekennzeichnet, daß die thermisch zersetzbare organische Verbindung ebenfalls eine organische Verbindung ist, die dem betreffenden Umwandlungsverfahren unterworfen wird.

9. Verfahren nach Anspruch 8, dadurch gekennzeichnet, daß als entsprechende Verbindung Toluol verwendet wird.

10. Verfahren nach den vorhergehenden Ansprüchen, dadurch gekennzeichnet, daß ZSM-5 als Zeolith verwendet wird.

11. Verfahren nach den vorhergehenden Ansprüchen, dadurch gekennzeichnet, daß der Zeolith eine Kristallgröße von mehr als 0,5 $\mu$m aufweist.

12. Verfahren nach den vorhergehenden Ansprüchen, dadurch gekennzeichnet, daß der Zeolith mit einem Bindemittel zusammengesetzt ist, z.B. Aluminiumoxid.

13. Verfahren nach den vorhergehenden Ansprüchen, dadurch gekennzeichnet, daß das Umwandlungsverfahren eine Alkylierung eines aromatischen Kohlenwasserstoffs, Disproportionierung eines alkylaromatischen Kohlenwasserstoffs, Xylolisomerisierung oder Umwandlung von niederen einwertigen Alkoholen und/oder deren entsprechenden Äthern in ein olefinreiches Kohlenwasserstoffgemisch ist.

**Revendications**

1. Procédé de régénération d'un catalyseur portant un dépôt de coke par suite de son emploi dans un procédé de conversion d'un composé organique, ce catalyseur comprenant une zéolite cristalline ayant un rapport de la silice à l'alumine d'au moins 12, un indice de contrainte de 1 à 12, une capacité de sorption du xylène d'au moins 1 g/100 g de zéolite et un temps de sorption de l'o-xylène pour 30% de ladite capacité supérieur à 10 min, cette capacité et ce temps de sorption étant mesurés à 120°C et sous une pression de xylène de 600 ± 106 Pa et ledit catalyseur ayant été modifié avant l'emploi par dépôt modéré sur celui-ci d'au moins 1% en poids de coke conférant une sélectivité, ce procédé comprenant l'exposition du catalyseur à une atmosphère contenant de l'hydrogène entre 425 et 650°C et entre 101 et 13 890 kPa pour éliminer le coke déposé par suite de l'emploi du catalyseur dans le procédé de conversion mais non le coke conférant la sélectivité.

2. Procédé selon la revendication 1, dans lequel ladite zéolite a été modifiée avant l'emploi par combinaison avec 0,5 à 40% en poids d'un oxyde d'antimoine, de bore, de magnésium et/ou de phosphore.

3. Procédé selon l'une des revendications 1 ou 2, dans lequel on régénère le catalyseur sous une pression de 170 à 6 996 kPa.

4. Procédé selon l'une quelconque des revendications précédentes, dans lequel on régénère le catalyseur sous une pression de 460 à 4 304 kPa.

5. Procédé selon l'une quelconque des revendications précédentes, dans lequel l'atmosphère contenant de l'hydrogène vient en contact avec le catalyseur à une vitesse spatiale pondérale horaire de 0,25 à 10.

6. Procédé selon l'une quelconque des revendications précédentes, dans lequel la durée de la régénération est de 1 à 48 h.

7. Procédé selon l'une quelconque des revendications précédentes, dans lequel on effectue le dépôt du coke conférant la sélectivité par contact du catalyseur avec un composé organique décomposable par la chaleur dans des conditions comprenant une température supérieure à la température de décomposition de ce composé mais inférieure à 648°C et un rapport hydrogène/composé compris entre 0 et 1.

8. Procédé selon la revendication 7, dans lequel ledit composé organique décomposable par la chaleur est également un composé organique soumis audit procédé de conversion.

9. Procédé selon la revendication 8, dans lequel ledit composé est le toluène.

10. Procédé selon l'une quelconque des revendications précédentes, dans lequel la zéolite est le ZSM-5.

11. Procédé selon l'une quelconque des revendications précédentes, dans lequel la zéolite a une taille cristalline supérieure à 0,5 $\mu$m.

12. Procédé selon l'une quelconque des revendications précédentes, dans lequel la zéolite est associée à un liant tel que l'alumine.

13. Procédé selon l'une quelconque des revendications précédentes, dans lequel ledit procédé de conversion est l'alkylation d'un hydrocarbure aromatique, la dismutation d'un hydrocarbure alkylaromatique, l'isomérisation du xylène ou la conversion de monoalcools inférieurs et/ou de leurs éthers en un mélange d'hydrocarbures riche en oléfines.

# FIGURE 1

# FIGURE 2

H₂ REGENERATION

TIME ON STREAM (MONTHS)